# EUROPEAN PATENT APPLICATION

(11) **EP 3 677 337 A1**
(43) Date of publication of application: **08.07.2020**
(21) Application number: 18852160.3
(22) Date of filing: 22.08.2018
(51) Int. Cl.: B03B 5/00, B03B 11/00, C12M 1/00, C12M 1/26

(54) **PARTICLE SEPARATION DEVICE AND PARTICLE SEPARATION APPARATUS USING SAME**

(30) Priority: 30.08.2017 JP 2017165703
(71) Applicant: Kyocera Corporation, Kyoto-shi, Kyoto-fu 612-8501 (JP)
(72) Inventor: YONETA, Masashi, Kyoto-shi Kyoto 612-8501 (JP); NAKAZONO, Jumpei, Kyoto-shi Kyoto 612-8501 (JP)
(74) Representative: Viering, Jentschura & Partner mbB Patent- und Rechtsanwälte
(86) International application number: PCT/JP2018/030940
(87) International publication number: WO 2019/044610

(57) **Abstract**

A particle separation device according to the present disclosure comprises a first flow path, a second flow path, and a plurality of branch flow paths. The first flow path includes a flow inlet (an opening). The second flow path is connected to the first flow path. Each of the plurality of branch flow paths is connected to the second flow path, and includes a flow outlet (an opening). The first flow path includes a bending unit, a first unit, and a second unit. In the bending unit, a direction in which a flow path extends is bent in a planar view. The first unit is located adjacent to the bending unit and closer to a flow inlet (an opening) than the bending unit in a planar view. The second unit is located adjacent to the bending unit and closer to a second flow path than the bending unit in a planar view. A width of the second unit in the first flow path is smaller than a width of the first unit in the first flow path in a planar view.

## Description

### Technical Field

The present disclosure relates to a particle separation device and a particle separation apparatus using the particle separation device.

### Background Art

Conventionally known is a particle separation device including a flow inlet and a plurality of flow outlets to separate and extract particles in a liquid using a minute flow path structure with a width of several µm to several hundred µm (microchannel) (refer to Patent Document 1, for example). In such a particle separation device, for example, when a liquid containing a plural types of particles (for example, blood) flows from the flow inlet, the plural type of particles (for example, red blood cells and white blood cells) can be separated and separately extracted from the plurality of flow outlets.

### Prior Art Documents

### Patent Documents

Japanese Patent Application Laid-Open No. 2012-76016

### Summary

A particle separation device according to the present disclosure comprises a first flow path, a second flow path, and a plurality of branch flow paths. The first flow path includes a flow inlet. The second flow path is connected to the first flow path. Each of the plurality of branch flow paths is connected to the second flow path, and includes a flow outlet. The first flow path includes a bending unit, a first unit, and a second unit. In the bending unit, a direction in which a flow path extends is bent in a planar view. The first unit is located adjacent to the bending unit and closer to the flow inlet than the bending unit in a planar view. The second unit is located adjacent to the bending unit and closer to the second flow path than the bending unit in a planar view. A width of the second unit in the first flow path is smaller than a width of the first unit in the first flow path in a planar view.

A particle separation apparatus according to the present disclosure comprises the particle separation device and a pump for flowing a liquid into the flow inlet.

### Brief Description of Drawings

[Fig. 1] A plan view schematically illustrating a first example of a particle separation device according to the present disclosure.
[Fig. 2] A plan view schematically illustrating a second example of the particle separation device according to the present disclosure.
[Fig. 3] A plan view schematically illustrating a third example of the particle separation device according to the present disclosure.
[Fig. 4] A plan view schematically illustrating a fourth example of the particle separation device according to the present disclosure.
[Fig. 5] A plan view schematically illustrating a fifth example of the particle separation device according to the present disclosure.
[Fig. 6] A plan view schematically illustrating a sixth example of the particle separation device according to the present disclosure.
[Fig. 7] A plan view schematically illustrating a seventh example of the particle separation device according to the present disclosure.
[Fig. 8] A diagram schematically illustrating an example of a particle separation apparatus according to the present disclosure.

### Description of Embodiment(s)

In a particle separation device described in Patent Document 1, a bypass flow path connecting an upstream and a downstream of a main flow path is made up, and a liquid flowing from an end portion on a downstream side of the bypass flow path into the main flow path presses particles in the liquid against one wall surface side in the main flow path, and flows the particles into a branch flow path opened in one wall surface of the main flow path, thereby separating the particles. However, there is a case where the particles in the liquid flow into the bypass flow path, and the bypass flow path gets clogged.

A particle separation device according to the present disclosure can reduce an occurrence of such a trouble. The particle separation device according to the present disclosure and a particle separation apparatus using the particle separation device are described in detail hereinafter.

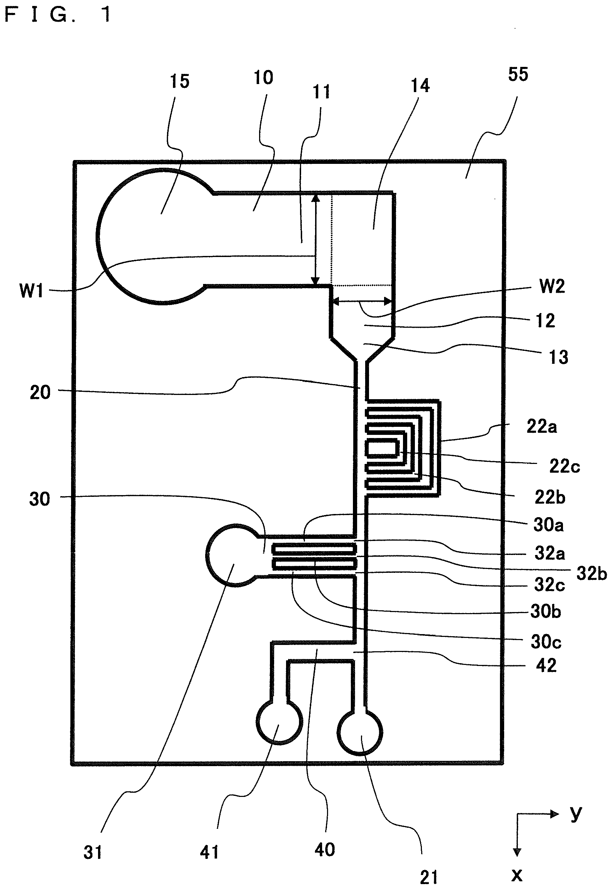

Fig. 1 is a plan view schematically illustrating a first example of the particle separation device according to the present disclosure. In Fig. 1, a line which indicates a hidden structure and thus should be originally indicated by a broken line is also indicated by a solid line for easily constructing the drawing.

The particle separation device according to the present disclosure includes a first flow path 10, a second flow path 20, and a plurality of branch flow paths (30, 40). The first flow path 10 includes a flow inlet (an opening 15). The second flow path 20 is connected to the first flow path 10. Each of the plurality of branch flow paths (30, 40) is connected to the second flow path 20, and includes a flow outlet (an opening 31 and an opening 41). The first flow path 10 includes a bending unit 14, a first unit 11, and a second unit 12. In the bending unit 14, a direction in which the flow path extends is bent in a planar view. The first unit 11 is located adjacent to the bending unit 14 and closer to the flow inlet (the opening 15) than the bending unit 14 in a planar view. The second unit 12 is located adjacent to the bending unit 14 and closer to the second flow path 20 than the bending unit 14 in a planar view. A width W2 of the second unit 12 in the first flow path 10 is smaller than a width W1 of the first unit 11 in the first flow path 10 in a planar view. This configuration constitutes a basic configuration of the particle separation device according to the present disclosure. The particle separation device according to the present disclosure needs to include this basic configuration, and the other configuration is not necessary, but can be appropriately changed. In Fig. 1, defined as the bending unit 14 is a portion located between two lines (indicated by dotted lines in Fig. 1) drawn in a width direction of the flow path (a direction perpendicular to the direction in which the flow path extends), the two lines starting from an angle on an inner side of a portion where the direction in which the flow path extends is bent in the first flow path 10, defined as the first unit 11 is a portion located adjacent to the bending unit 14 and closer to the flow inlet (the opening 15) than the bending unit 14 and having a constant width in a planar view, and defined as the second unit 12 is a portion located adjacent to the bending unit 14 and closer to the second flow path 20 than the bending unit 14 and having a constant width in a planar view.

According to this basic configuration, particles in a liquid can be brought to one side of a horizontal section of the flow path in the second unit 12 in the first flow path 10 and the second flow path 20, thus the particles can be subsequently separated easily.

The first example of the particle separation device according to the present disclosure is described in detail hereinafter. The first flow path 10, the second flow path 20, and the branch flow path (30, 40) are formed in a substrate 55. The substrate 55 can be formed using various types of material. For example, various types of resin material such as acrylic, polycarbonate, and dimethylpolysiloxane (PDMS), an inorganic material such as glass, silicon, and ceramic, and various types of metal material such as stainless can be used as the material of the substrate 55. A plural types of material may be used in combination. The substrate 55 is preferably made up of a transparent material by reason that the liquid flowing in the flow path in the substrate 55 can be recognized from outside.

For example, two substrates, one or both of which include a groove or a hole, are bonded directly or using an adhesive agent, thus the substrate 55 including the first flow path 10, the second flow path 20, and the branch flow path (30, 40) can be easily manufactured. A conventionally-known technique such as wet etching, dry etching, laser processing, and mechanical processing, for example, can be used for forming the groove or the hole. A cross-sectional shape of each of the first flow path 10, the second flow path 20, and the branch flow path (30, 40) can be a rectangular shape, for example, however, there may also be a portion having a cross-sectional shape other than the rectangular shape. A depth of the flow path is preferably constant, however, it is also applicable that there is a portion locally having a slightly different depth. The depths of the first flow path 10, the second flow path 20, and the branch flow path (30, 40) can be set in accordance with sizes of the particles to be separated and the like, for example, and can be set to 10 µm to 30 µm, for example. The widths of the first flow path 10, the second flow path 20, and the branch flow path (30, 40) can be set in accordance with sizes of the particles to be separated, and can be set to several µm to several hundred µm, for example.

The first flow path 10 includes the opening 15, the bending unit 14, the first unit 11, the second unit 12, and a third unit 13. The opening 15 is a flow inlet into which the liquid flows from outside, and is opened on an upper surface of the substrate 55.

In the bending unit 14, the direction in which the flow path extends is bent in a planar view. In the present example, in the bending unit 14, the direction in which the flow path extends is bent at a 90-degree angle from a y direction to an x direction. The bending angle in the bending unit 14 can be set to substantially 90 degrees (85 to 95 degrees), for example, however, the other angle is also applicable.

The first unit 11 is a portion located adjacent to the bending unit 14 and closer to the opening 15 than the bending unit 14 in a planar view. The width W1 of the first unit 11 may be set to approximately 0.4 to 1.6 mm, for example. It is also applicable that there is a portion having a different width between the first unit 11 and the opening 15. A length between the opening 15 and the bending unit 14 may be set to approximately 2 to 10 mm, for example.

The second unit 12 is a portion located adjacent to the bending unit 14 and closer to the second flow path 20 than the bending unit 14 in a planar view. The width W2 of the second unit 12 is approximately 0.2 to 0.8 mm, for example, and can be set to be smaller than the width W1 of the first unit 11.

The third unit 13 is a portion whose width decreases from an upstream side toward a downstream side in a planar view. The third unit 1133 is a portion located on an end portion on a downstream side of the first flow path 10, and the second flow path 20 is connected to an end portion on a downstream side (a +x direction side in Fig. 1) of the third unit 13. The first flow path 10 includes the third unit 13, thereby being able to make the flow of the liquid from the first flow path 10 to the second flow path 20 smooth, however, the third unit 13 may not be included in some cases.

The second flow path 20 is connected to the first flow path 10, and includes an opening 21. The opening 21 is opened in a lower surface of the substrate 55. The opening 21 is provided on an end portion on a downstream side of the second flow path 20, and a liquid which has not flowed into the branch flow path (30, 40) in a liquid which has flowed from the first flow path 10 flows out from the opening 21.

The particle separation device in the present example includes a plurality of bypass flow paths (22a, 22b, 22c) connected to the second flow path 20. Fig. 1 illustrates an example of including the three bypass flow paths (22a, 22b, 22c) for simplifying the illustration, however, approximately 10 to 30 bypass flow paths, for example, can be actually formed.

Each of the bypass flow paths (22a, 22b, 22c) includes two connection ports opened in an inner wall surface of the second flow path 20, and are connected to the second flow path 20 via the two connection ports. One of the two connection ports is located on an upstream side (the opening 15 side) of the second flow path 20, and the other one of the two connection ports is located on the downstream side (the opening 21 side) of the second flow path 20. A size of a cross section of the connection port located on the opening 15 side is preferably smaller than sizes of the particles to be separated, and in such a case, the flow of the particles into the bypass flow paths (22a, 22b, 22c) can be reduced. In each of the bypass flow paths (22a, 22b, 22c), the liquid flows from the connection port located on the opening 15 side thereinto, and the liquid flows out from the connection port located on the opening 21 side to the second flow path 20. Accordingly, the particles in the liquid flowing in the second flow path 20 can be pressed against a wall surface (a wall surface on a -y direction side in Fig. 1) on an opposite side of the bypass flow path (22, 22b, 22c) in the second flow path 20.

The present example describes the example of including the plurality of bypass flow paths (22a, 22b, 22c), however, the plurality of bypass flow paths (22a, 22b, 22c) may not be included in some cases. One or more differences in level or protrusions may be provided in the inner wall of the second flow path 20, for example, to control positions where the particles flow in the horizontal section of the second flow path 20.

One end of the branch flow path 30 is branched into a plurality of flow paths (30a, 30b, 30c), and each of the plurality of flow paths (30a, 30b, 30c) is connected to the second flow path 20. The flow path 30a includes a connection port 32a opened in the inner wall surface of the second flow path 20, and is connected to the second flow path 20 via the connection port 32a. The flow path 30b includes a connection port 32b opened in the inner wall surface of the second flow path 20, and is connected to the second flow path 20 via the connection port 32b. The flow path 30c includes a connection port 32c opened in the inner wall surface of the second flow path 20, and is connected to the second flow path 20 via the connection port 32c.

The branch flow path 30 includes the opening 31 on the other end. The opening 31 is a flow outlet from which the liquid flows out, and is opened in the lower surface of the substrate 55. Then, the liquid flowing from the second flow path 20 via the plurality of connection ports (32a, 32b, 32c) flows outside from the opening 31.

Fig. 1 illustrates the example that the one end of the branch flow path 30 is branched into the three flow paths (30a, 30b, 30c) for simplifying the illustration, however, the branch flow path 30 can be branched into more flow paths as usage, thus can be branched into approximately 10 to 30 flow paths, for example.

The branch flow path 40 includes the opening 41 and a connection port 42. The connection port 42 is opened in the inner wall surface of the second flow path 20, and the branch flow path 40 and the second flow path 20 are linked to each other via the connection port 42. The opening 41 is a flow outlet from which the liquid flows out, and is opened in the lower surface of the substrate 55. Then, the liquid flowing from the second flow path 20 via the connection port 42 flows outside from the opening 41.

The connection port 32a, the connection port 32b, the connection port 32c, and the connection port 42 are provided on a wall surface on an opposite side of the bypass flow paths (22a, 22b, 22c) in the second flow path 20, that is to say, a wall surface to which the particles flow closely in the second flow path 20. The connection port 32a, the connection port 32b, the connection port 32c, and the connection port 42 are provided side by side along a direction in which the liquid flow. The connection port 42 is located closer to a downstream side (a side farther away from the opening 15 which is the flow inlet) than the plurality of connection ports (the connection port 32a, the connection port 32b, and the connection port 32c) included in the branch flow path 30.

Sizes of the openings of the plurality of connection ports (the connection port 32a, the connection port 32b, and the connection port 32c) included in the branch flow path 30 are set so that a smallest one of the particles to be separated flows into the branch flow path 30 and a particle larger than the smallest one hardly flows thereinto. Thus, the smallest one of the particles to be separated and extracted flows from the connection port 32a, the connection port 32b, and the connection port 32c into the branch flow path 30, and flows outside from the opening 31 which is the flow outlet. In this manner, the smallest one of the particles to be separated can be extracted from the opening 31.

For example, when red blood cells (erythrocyte) and white blood cells (leukocyte) in blood are separated and extracted, the red blood cells are smaller than the white blood cells, thus the red blood cells flow into the branch flow path 30 from the connection port 32a, the connection port 32b, and the connection port 32c, and flows outside from the opening 31. Accordingly, the red blood cells can be extracted from the opening 31. In this case, the size of each of the connection port 32a, the connection port 32b, and the connection port 32c can be set to approximately 15 µm, for example.

The size of the opening in the connection port 42 can be set so that the second smallest one of the particles to be separated flows into the branch flow path 40 and a particle larger than the second smallest one hardly flows thereinto. Thus, the second smallest one of the particles to be separated and extracted flows from the connection port 42 into the branch flow path 40, and flows outside from the opening 41 which is the flow outlet. In this manner, the second smallest one of the particles to be separated can be extracted from the opening 41. For example, when red blood cells and white blood cells in blood are separated and extracted, the white blood cells are larger than the red blood cells, thus the white blood cells flow into the branch flow path 40 from the connection port 42, and flows outside from the opening 41. Accordingly, the white blood cells can be extracted from the opening 41. In this case, the size of the connection port 42 can be set to approximately 20 µm, for example.

The opening 21 is located closer to the downstream side (the side farther away from the opening 15 which is the flow inlet) than the connection port 42, and is opened in the lower surface of the substrate 55. The remaining liquid, which has not flowed into the branch flow path 30 and the branch flow path 40, in the liquid which has flowed into the second flow path 20 flows outside from the opening 21. For example, when the red blood cells and the white blood cells in the blood are separated and extracted, remaining components in which almost all of the red blood cells and white blood cells are removed from the blood can be extracted from the opening 21 which is the flow outlet.

For example, when the red blood cells and the white blood cells in the blood are separated and extracted, there is variations in sizes of the red blood cells and sizes of the white blood cells, so that there is a case where a white blood cell having a smaller size flows into the branch flow path 30 via the connection port 32a, the connection port 32b, and the connection port 32c being the smallest ones and is extracted from the flow outlet (the opening 31) with the red blood cells. In contrast, there is a case where the red blood cell is mixedly included in the liquid which has flowed out from the flow outlet (the opening 41) of the branch flow path 40. However, the number of red blood cells in the liquid flowing out from the flow outlet (the opening 31) of the branch flow path 30 is much larger than the number of white blood cells, and the number of red blood cells in the liquid flowing out from the flow outlet (the opening 41) of the branch flow path 40 is much larger than the number of white blood cells. "The separation" in the present disclosure means that such a state can be achieved, and does not mean that the separation can be performed with no mixing.

In this manner, the liquid flowing into the flow inlet (the opening 15) flows out from the plurality of flow outlets (the opening 31, the opening 41, and the opening 21), and the particles in the liquid can be separated and extracted from the different flow outlets.

It is applicable in the particle separation device according to the present disclosure, as the present example, that the first flow path 10 includes the third unit 13 whose width decreases from the upstream side toward the downstream side in a planar view, and the second flow path 20 is connected to the end portion on the downstream side of the third unit 13. Such a configuration can make the flow of the liquid from the first flow path 10 to the second flow path 20 smooth.

It is also applicable in the particle separation device according to the present disclosure, as the present example, that the direction in which the liquid flows is bent to a right side of the first unit 11 in the bending unit 14 relative to the direction from the upstream side toward the downstream side (a +y direction in Fig. 1), and the branch flow path (30, 40) is connected on the right side of the second flow path 20 relative to the direction from the upstream side toward the downstream side (the +x direction in Fig. 1) in a planar view. According to such a configuration, the particles in the liquid can be brought to the side to which the branch flow path (30, 40) is connected in the second flow path 20, thus a separation efficiency of the particles can be increased.

It is also applicable in the particle separation device according to the present disclosure, as the present example, that the bending unit 14 is bent to the right side of the first unit 11 relative to the direction from the upstream side toward the downstream side (a +y direction in Fig. 1), and the branch flow path (30, 40) is connected on the left side of the second flow path 20 relative to the direction from the upstream side toward the downstream side (the +x direction in Fig. 1) in a planar view. According to such a configuration, the particles in the liquid can be brought to the side opposite to the side to which the bypass flow path (22a, 22b, 22c) is connected in the second flow path 20 (the - y direction side in Fig. 1), thus the flow of the particles in the liquid into the bypass flow path (22a, 22b, and 22c) can be reduced.

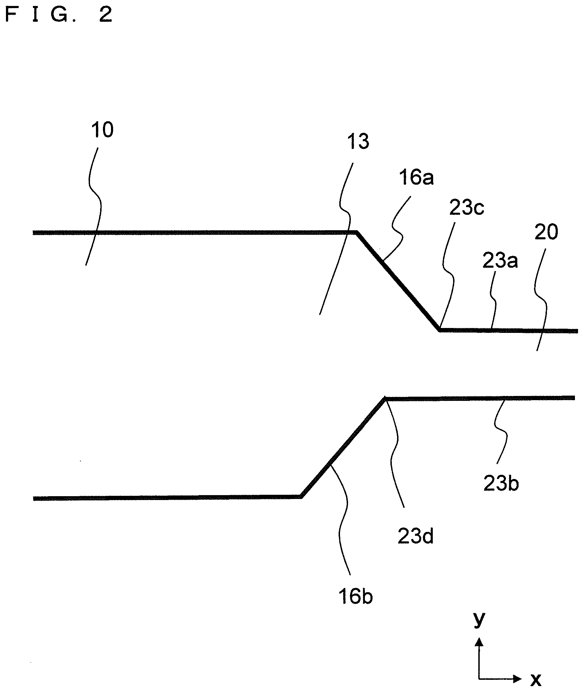

Next, a second example of the particle separation device according to the present disclosure is described. Fig. 2 is a plan view schematically illustrating the second example of the particle separation device according to the present disclosure, and is a drawing in which only a portion around a connection part between the third unit 13 and the second flow path 20 is enlarged. A configuration different from that in the first example is described in the present example. The same reference signs will be assigned to the same configuration, and a repetitive description is omitted.

In the present example, as illustrated in Fig. 2, a portion 23c where an inner wall surface 23a on a left side of the second flow path 20 relative to the direction from the upstream side toward the downstream side and an inner wall surface 16a of the first flow path 10 are in contact with each other is located closer to the downstream side (the side farther away from the opening 15 which is the flow inlet and the +x direction side in Fig. 1 and Fig. 2) than a portion 23d where an inner wall surface 23b on a right side of the second flow path 20 relative to the direction from the upstream side toward the downstream side and an inner wall surface 16b of the first flow path 10 are in contact with each other in a planar view. According to such a configuration, the particles in the liquid can be further brought to the side to which the branch flow path (30, 40) is connected in the second flow path 20. A distance from the portion 23c where the inner wall surface 23a of the second flow path 20 and the inner wall surface 16a of the first flow path 10 are in contact with each other to the portion 23d where the inner wall surface 23b of the second flow path 20 and the inner wall surface 16b of the first flow path 10 are in contact with each other along the direction from the upstream side toward the downstream side in the second flow path 20 (the +x direction in Fig. 1 and Fig. 2) can be appropriately set. It is applicable that this distance is set to twice a dimension of the second flow path 20 in a width direction (the y direction in Fig. 1 and Fig. 2) or more, for example.

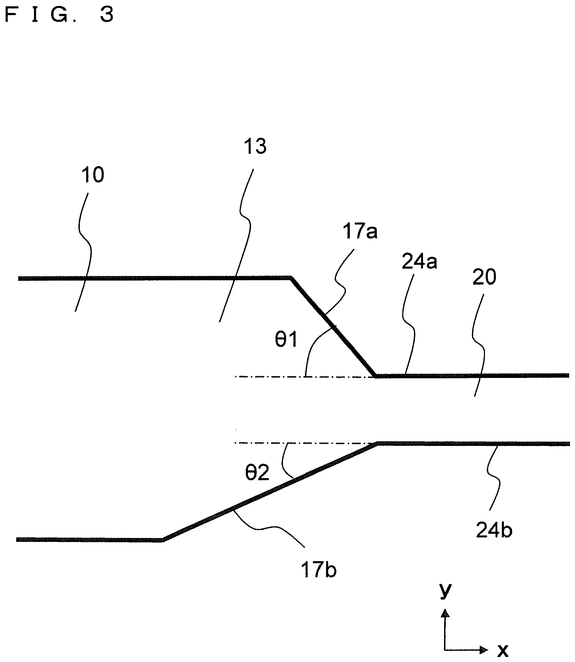

Next, a third example of the particle separation device according to the present disclosure is described. Fig. 3 is a plan view schematically illustrating the third example of the particle separation device according to the present disclosure, and is a drawing in which only a portion around a connection part between the third unit 13 and the second flow path 20 is enlarged. A configuration different from that in the first example is described in the present example. The same reference signs will be assigned to the same configuration, and a repetitive description is omitted.

In the present example, as illustrated in Fig. 3, an angle θ1 between an inner wall surface 24a on a left side of the second flow path 20 relative to the direction from the upstream side toward the downstream side and an inner wall surface 17a of the first flow path 10 in contact with the inner wall surface 24a on the left side is larger than an angle θ2 between an inner wall surface 24b on a right side of the second flow path 20 relative to the direction from the upstream side toward the downstream side and an inner wall surface 17b of the first flow path 10 in contact with the inner wall surface 24b on the right side in a planar view. According to such a configuration, the particles in the liquid can be further brought to the side to which the branch flow path (30, 40) is connected in the second flow path 20. The angle θ1 and the angle θ2 can be appropriately set, and θ1 can be set to approximately 1.5 to 3 times as large as θ2, for example. θ1 can be set to approximately 60° to 90°, for example, and θ2 can be set to approximately 30° to 60°, for example.

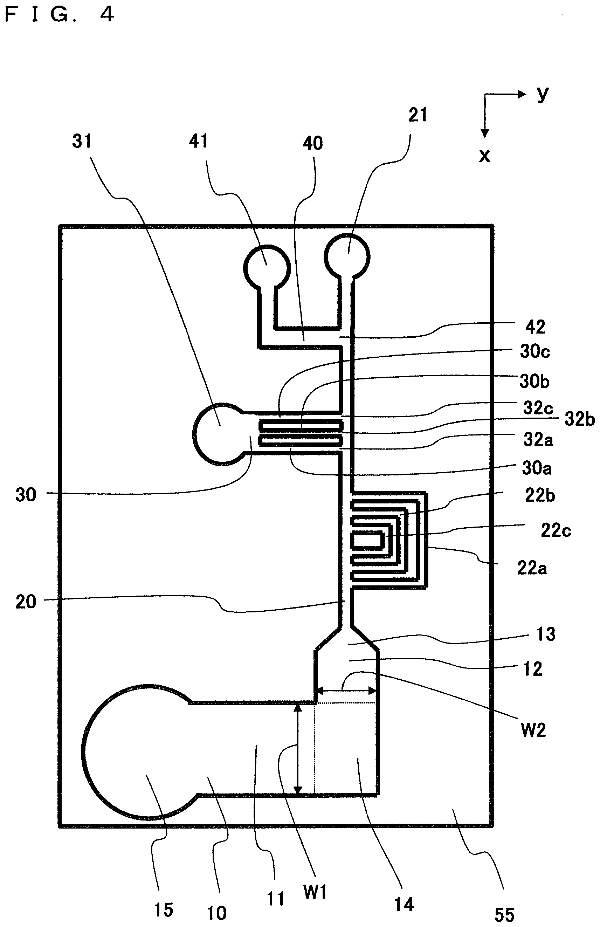

Next, a fourth example of the particle separation device according to the present disclosure is described. Fig. 4 is a plan view schematically illustrating the fourth example of the particle separation device according to the present disclosure. A configuration different from that in the first example is described in the present example. The same reference signs will be assigned to the same configuration, and a repetitive description is omitted.

In the present example, as illustrated in Fig. 4, the bending unit 14 is configured to be bent to a left side of the first unit 11 relative to the direction from the upstream side toward the downstream side (the +y direction in Fig. 4), and the branch flow path (30, 40) is connected on the left side of the second flow path 20 relative to the direction from the upstream side toward the downstream side (the -x direction in Fig. 4) in a planar view. According to such a configuration, the particles in the liquid can be brought to the side to which the branch flow path (30, 40) is connected in the second flow path 20, thus the separation efficiency of the particles can be increased.

In the present example, as illustrated in Fig. 4, in the bending unit 14, the direction in which the liquid flows is bent to the left side of the first unit 11 relative to the direction from the upstream side toward the downstream side (the +y direction in Fig. 4), and the bypass flow path (22a, 22b, and 22c) is connected on the right side of the second flow path 20 relative to the direction from the upstream side toward the downstream side (the -x direction in Fig. 4) in a planar view. According to such a configuration, the particles in the liquid can be brought to the side opposite to the side to which the bypass flow path (22a, 22b, and 22c) is connected in the second flow path 20 (the -y direction side in Fig. 4), thus the flow of the particles in the liquid into the bypass flow path (22a, 22b, and 22c) can be reduced.

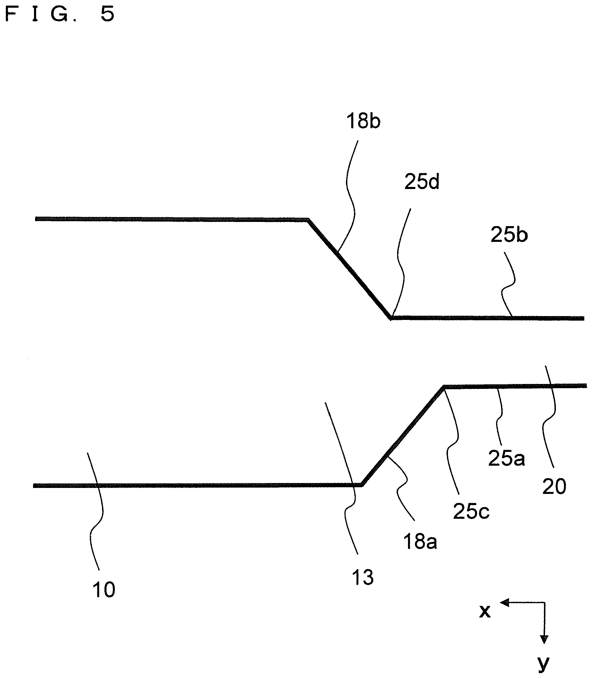

Next, a fifth example of the particle separation device according to the present disclosure is described. Fig. 5 is a plan view schematically illustrating the fifth example of the particle separation device according to the present disclosure, and is a drawing in which only a portion around a connection part between the third unit 13 and the second flow path 20 is enlarged. A configuration different from that in the fourth example is described in the present example. The same reference signs will be assigned to the same configuration, and a repetitive description is omitted.

In the present example, as illustrated in Fig. 5, a portion 25c where an inner wall surface 25a on a right side of the second flow path 20 relative to the direction from the upstream side toward the downstream side and an inner wall surface 18a of the first flow path 10 are in contact with each other is located closer to the downstream side (the side farther away from the opening 15 which is the flow inlet and the -x direction side in Fig. 4 and Fig. 5) than a portion 25d where an inner wall surface 25b on a left side of the second flow path 20 relative to the direction from the upstream side toward the downstream side and an inner wall surface 18b of the first flow path 10 are in contact with each other in a planar view. According to such a configuration, the particles in the liquid can be further brought to the side to which the branch flow path (30, 40) is connected in the second flow path 20. A distance from the portion 25c where the inner wall surface 25a of the second flow path 20 and the inner wall surface 18a of the first flow path 10 are in contact with each other to the portion 25d where the inner wall surface 25b of the second flow path 20 and the inner wall surface 18b of the first flow path 10 are in contact with each other along the direction from the upstream side toward the downstream side in the second flow path 20 (the -x direction in Fig. 4 and Fig. 5) can be appropriately set. It is applicable that this distance is set to twice a dimension of the second flow path 20 in the width direction (the y direction in Fig.4 and Fig. 5) or more, for example.

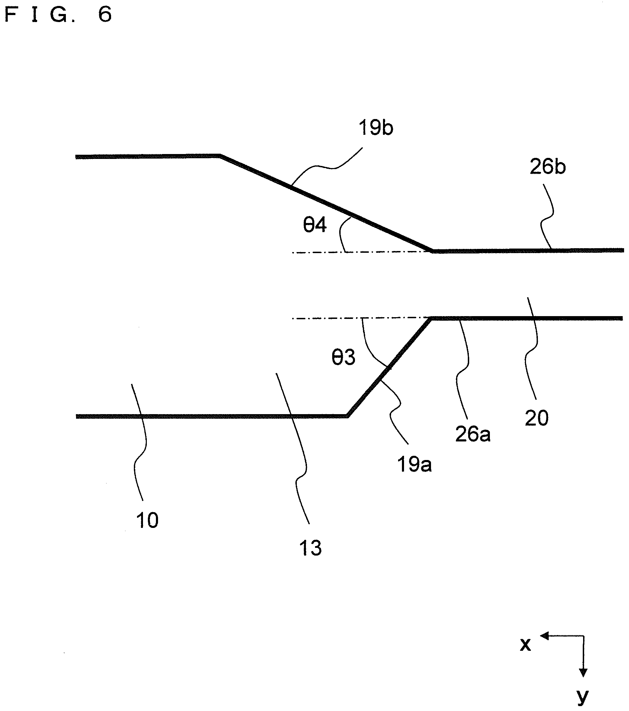

Next, a sixth example of the particle separation device according to the present disclosure is described. Fig. 6 is a plan view schematically illustrating the sixth example of the particle separation device according to the present disclosure, and is a drawing in which only a portion around a connection part between the third unit 13 and the second flow path 20 is enlarged. A configuration different from that in the fourth example is described in the present example. The same reference signs will be assigned to the same configuration, and a repetitive description is omitted.

In the present example, as illustrated in Fig. 6, an angle θ3 between an inner wall surface 26a on a right side of the second flow path 20 relative to the direction from the upstream side toward the downstream side and an inner wall surface 19a of the first flow path 10 in contact with the inner wall surface 26a on the right side is larger than an angle θ4 between an inner wall surface 26b on a left side of the second flow path 20 relative to the direction from the upstream side toward the downstream side and an inner wall surface 19b of the first flow path 10 in contact with the inner wall surface 26b on the left side in a planar view. According to such a configuration, the particles in the liquid can be further brought to the side to which the branch flow path (30, 40) is connected in the second flow path 20. The angle θ3 and the angle θ4 can be appropriately set, and θ3 can be set to approximately 1.5 to 3 times as large as θ4, for example. θ3 can be set to approximately 60° to 90°, for example, and θ4 can be set to approximately 30° to 60°, for example.

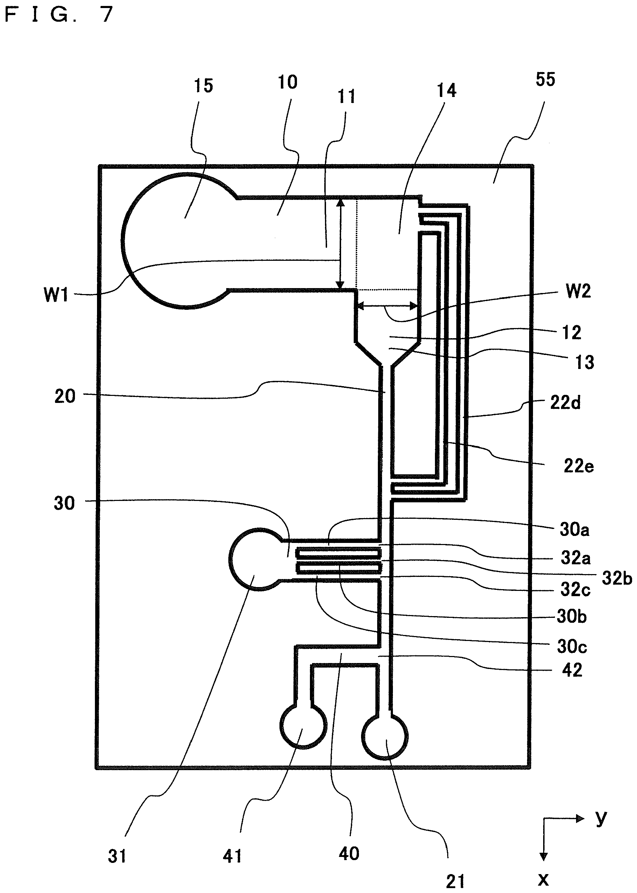

Next, a seventh example of the particle separation device according to the present disclosure is described. Fig. 7 is a plan view schematically illustrating the seventh example of the particle separation device according to the present disclosure. A configuration different from that in the first example is described in the present example. The same reference signs will be assigned to the same configuration, and a repetitive description is omitted.

In the present example, as illustrated in Fig. 7, the particle separation device includes bypass flow paths (22d, 22e) instead of the bypass flow paths (22a, 22b, 22c), and a connection port located on the upstream side (the side closer to the opening 15 which is the flow inlet) in two connection ports included in each of the bypass flow paths (22d, 22e) is opened in an inner wall surface near a corner on an outer side of the bending unit 14. A connection port located on the downstream side (the side farther away from the opening 15 which is the flow inlet) in the two connection ports included in each of the bypass flow paths (22d, 22e) is opened in the inner wall surface of the second flow path 20 in the manner similar to the first example described above. That is to say, each of the bypass flow paths (22d, 22e) connects a portion near the corner on the outer side of the bending unit 14 and a portion located halfway of the second flow path 20. In the bending unit 14, the particles in the liquid are brought to the inner side and flow therein, and there is almost no particle near the corner on the outer side of the bending unit 14, thus the configuration described in the present example can further reduce the particles flowing into the bypass flow paths (22d, 22e) and causing the clog in the bypass flow paths (22d, 22e).

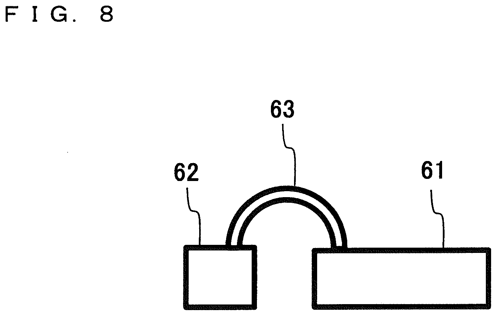

Next, a particle separation apparatus according to the present disclosure is described. Fig. 8 is a diagram schematically illustrating an example of a particle separation apparatus according to the present disclosure. The particle separation apparatus according to the present disclosure includes a particle separation device 61 and a pump 62. The particle separation device 61 is the particle separation device in the first example described above, and a flow inlet (the opening 15) in the particle separation device 61 and the pump 62 are connected by a tube 63. A liquid sent from the pump 62 flows into the flow inlet (the opening 15) in the particle separation device 61 through the tube 63.

The pump 62 is used for flowing a fluid (a liquid) including particles into the flow inlet (the opening 15) of the particle separation device 61, and known various pumps can be applied thereto. The pump 62 preferably has a function of flowing a small amount of liquid including particles into the flow inlet (the opening 15) of the particle separation device 61 at a constant flow rate. A syringe pump, for example, can be preferably applied to the pump 62, however, the other pump such as an electroosmotic flow pump, a peristaltic pump, and a gas pump are also applicable.

The tube 63 can be made up using a tube made of known various materials in accordance with a liquid to be used. A silicone tube, for example, can be preferably applied to the tube 63. The tube 63 is not necessary, thus the particle separation apparatus needs not include the tube 63 when the particle separation device 61 and the pump 62 are directly connected to each other, for example.

The particle separation device and the particle separation apparatus according to the present disclosure are not limited to those in the plurality of examples described above, however, various modifications are applicable. For example, a configuration made up of the plurality of configurations described in the different examples is also applicable.

### Explanation of Reference Signs

10: first flow path
11: first unit
12: second unit
13: third unit
14: bending unit
15: flow inlet (opening)
21, 31, 41: flow outlet (opening)
20: second flow path
22a, 22b, 22c, 22d, 22e: bypass flow path
30, 40: branch flow path
61: particle separation device
62: pump

## Claims

1. A particle separation device, comprising:
a first flow path including a flow inlet;
a second flow path connected to the first flow path; and
a plurality of branch flow paths each connected to the second flow path and each including a flow outlet, wherein
in a planar view,
the first flow path includes a bending unit in which a direction in which the flow path extends is bent, a first unit located adjacent to the bending unit and closer to the flow inlet than the bending unit, and a second unit located adjacent to the bending unit and closer to the second flow path than the bending unit, and
a width of the second unit in the first flow path is smaller than a width of the first unit in the first flow path.

2. The particle separation device according to claim 1, wherein
in a planar view,
the first flow path includes a third unit whose width decreases from an upstream side toward a downstream side, and the second flow path is connected to an end portion on a downstream side of the third unit.

3. The particle separation device according to claim 1 or 2, wherein
in a planar view,
the bending unit is configured to be bent to a right side of the first unit relative to a direction from an upstream side toward a downstream side, and
the branch flow path is connected on a right side of the second flow path relative to a direction from an upstream side toward a downstream side.

4. The particle separation device according to claim 3, wherein
in a planar view,
a portion where an inner wall surface on a left side of the second flow path relative to a direction from an upstream side toward a downstream side and an inner wall surface of the first flow path are in contact with each other is located closer to a downstream side than a portion where an inner wall surface on a right side of the second flow path relative to a direction from an upstream side toward a downstream side and an inner wall surface of the first flow path are in contact with each other.

5. The particle separation device according to claim 3 or 4, wherein
in a planar view,
an angle between an inner wall surface on a left side of the second flow path relative to a direction from an upstream side toward a downstream side and an inner wall surface of the first flow path in contact with the inner wall surface on the left side is larger than an angle between an inner wall surface on a right side of the second flow path relative to a direction from an upstream side toward a downstream side and an inner wall surface of the first flow path in contact with the inner wall surface on the right side.

6. The particle separation device according to claim 1 or 2, wherein
in a planar view,
the bending unit is configured to be bent to a left side of the first unit relative to a direction from an upstream side toward a downstream side, and
the branch flow path is connected on a left side of the second flow path relative to a direction from an upstream side toward a downstream side.

7. The particle separation device according to claim 6, wherein
in a planar view,
a portion where an inner wall surface on a right side of the second flow path relative to a direction from an upstream side toward a downstream side and an inner wall surface of the first flow path are in contact with each other is located closer to a downstream side than a portion where an inner wall surface on a left side of the second flow path relative to a direction from an upstream side toward a downstream side and an inner wall surface of the first flow path are in contact with each other.

8. The particle separation device according to claim 6 or 7, wherein
in a planar view,
an angle between an inner wall surface on a right side of the second flow path relative to a direction from an upstream side toward a downstream side and an inner wall surface of the first flow path in contact with the inner wall surface on the right side is larger than an angle between an inner wall surface on a left side of the second flow path relative to a direction from an upstream side toward a downstream side and an inner wall surface of the first flow path in contact with the inner wall surface on the left side.

9. A particle separation apparatus, comprising:
the particle separation device according to any one of claims 1 to 8; and
a pump for flowing a fluid into the flow inlet.
